# EUROPEAN PATENT APPLICATION

(11) **EP 4 726 041 A1**
(43) Date of publication of application: **15.04.2026**
(21) Application number: 24383109.6
(22) Date of filing: 11.10.2024
(51) Int. Cl.: C12N 15/113, C12N 9/22

(54) **MED12 INHIBITOR, OR PHARMACEUTICAL COMPOSITION COMPRISING THEREOF, FOR USE IN A METHOD FOR THE TREATMENT OF CANCER**

(71) Applicant: Instituto de Investigación Biomédica de Salamanca De la Fundación Instituto Ciencia de la Salud De Castilla y León, 37007 Salamanca (ES); Universidad De Salamanca, 37008 Salamanca (ES)
(72) Inventor: GONZÁLEZ SARMIENTO, Rogelio, 37007 Salamanca (ES); GESTOSO UZAL, Nerea, 37007 Salamanca (ES); MOLINERO SICILIA, Laura, 37007 Salamanca (ES)
(74) Representative: Hoffmann Eitle

(57) **Abstract**

The present invention refers to Mediator complex subunit 12 (MED 12) inhibitors, or pharmaceutical composition comprising thereof, for use in a method for the treatment of cancer.

## Description

### FIELD OF THE INVENTION

The present invention refers to the medical field. Particularly, the present invention refer to Mediator complex subunit 12 (MED12) inhibitors, or pharmaceutical composition comprising thereof, for use in a method for the treatment of cancer.

### STATE OF THE ART

Head and neck cancer groups have an estimated incidence of 600,000 new cases per year worldwide and a mortality rate of 40-50%. Head and neck squamous cell carcinomas (HNSCC) account for 90% of all head and neck cancers developing in the epithelial cells of the mucous linings. This entity can be detected at several anatomic localizations including the paranasal sinuses, nasal cavity, oral cavity, tongue, pharynx, larynx, and salivary glands; with the latter three being the most common. Despite originating from the same type of cell, HNSCC is a highly heterogenous cancer from both clinical-pathological and molecular perspective, making it challenging to consider it as a single entity. Such heterogeneity is mainly associated with the different etiologies of the carcinoma, the large number of genetic alterations that promote or arise from the tumor, and the complexity of the anatomical structures in which it develops.

The etiology of HSNCC is classically associated with risk factors such as tobacco and alcohol, in a dose-dependent manner. Approximately 75% of HNSCCs are associated with these habits, highlighting their synergist effect. Additionally, areca nut consumption poses a risk. These environmental risk factors are associated with clinically challenging tumours that exhibit a high rate of molecular alterations.

Recently, infections of papillomavirus (HPV) and Epstein-Barr virus (EBV) have emerged as major etiologic factors for HNSCC. Both infections can cause precancerous lesions in mucosal linings that can progress to invasive cancers. HPV, particularly types 16, 18, and 45, is primarily linked to oropharyngeal carcinomas and is associated with a better overall prognosis. EBV is recognized as a primary etiologic factor in nasopharyngeal carcinoma.

Lastly, another potential, although less common, cause of HNSCC tumor development is primarily based on cellular aging. These tumours are HPV and EBV negative but differ from those linked to other risk factors by presenting a significantly smaller number of mutations.

In general terms, carcinogenesis is the process through which cancer develops, typically divided into three phases: mutation, promotion, and progression. HNSCC initially arises from genetic or epigenetic alterations in the aerodigestive tract, leading to a tumoral phenotype. Subsequently, during promotion and progression towards tumor formation, there is uncontrolled cell proliferation (hyperplasia) and alterations in cell shape (dysplasia), resulting in carcinoma in situ and ultimately invasive carcinoma. It has been described that the severity of dysplastic changes is directly correlated with the quantity of genetic alterations. Additionally, it is hypothesized that carcinogenesis likely begins with the accumulation of mutations in a single adult stem cell, whose progeny proliferates.

The mentioned alterations result in the inactivation of tumor suppressor genes, activation of proto-oncogenes, or both at the same time. In early stages, common deletions involve 9p21, 3p14.2, and loss-of-function mutations in TP53, while more advanced stages are often characterized by deletions in 8p, 18q, or 3p24-3p26.

It is also noted that tumoral development from the mentioned alterations depends on its etiology. In HPV-positive cases, the expression of E6 and E7 genes, known to reduce the levels of the host cell tumor suppressor proteins p53 and pRb respectively, play crucial roles in the oncogenic transformation of virus-infected cells, whereas in HPV-negative cases, the spectrum of mutations influencing carcinogenesis is much broader. The most frequently mutated genes described until date are tumor suppressors such as TP53, CDKN2A, EGFR, FAT1, NOTCH1, KMT2D, NSD1 and GFBR2. However, many of these mutations occur at low frequencies, and their functional consequences remain unclear4. Besides mutations, genes may be activated by amplification or inactivated by losses or epigenetic changes.

Most HNSCC patients are diagnosed at a locally advanced stage, primarily due to the unnoticeable nature of the lesion and atypical early symptoms. Additionally, a single treatment method often proves insufficient, necessitating a multidisciplinary tailored to the individual patient's condition and disease stage. In early stages, the recommended treatment typically involves local interventions such as surgery or radiation therapy alone, while more advanced stages require systemic treatments like chemotherapy or targeted therapy. Chemoradiation is often preferred in cases where surgery is not an option. Also, induction chemotherapy (chemotherapy before other treatments) may be used to shrink the tumor before surgery or radiation therapy.

The standard chemotherapeutic agents in HNSCC clinical practice are taxanes (paclitaxel/docetaxel), cisplatin, and 5-fluorouracil (5-FU). The administration of these drugs in combined or intensified regimens aims to achieve synergy among different mechanisms of action to promote patient response. Taxanes induce mitotic blockade, preventing cell division and promoting apoptosis. Their mechanism of action involves binding to the β-subunit of microtubules, promoting tubulin assembly, and blocking division during metaphase. Cisplatin's primary target is the DNA of cells, to which it binds, causing lethal damage. Additionally, it can bind to other biomolecules such as glutathione oligopeptide (GSH) or metallothioneins (MTs), generating reactive oxygen species (ROS), which trigger mitochondrial membrane permeabilization and cause further DNA damage. Lastly, 5-FU is an antimetabolite drug that erroneously incorporates primary antimetabolites into DNA and RNA causing damage and apoptosis20.

So, there is an unmet medical need to find reliable therapeutic target aimed a treating cancer, particularly solid tumours like HNSCC or ovarian cancer. The present invention is focused on solving this problem and an innovative therapeutic strategy is herein provided.

### Description of the invention

### Brief description of the invention

The present invention refers to MED12 inhibitors, or pharmaceutical composition comprising thereof, for use in a method for the treatment of cancer.

Specifically, the present invention aims at elucidating the role of the MED12 gene in the pathogenesis of HNSCC and determining the potential therapeutic implications of targeting MED12 in these cancers. The inventors of the present invention have demonstrated that the inactivation of the MED 12 gene control tumor proliferation proposing a potential therapeutic approach in HNSCC. In order to do so, the following objectives were established for this study:
- Generate MED12-deficient *in vitro* HNSCC models by using CRISPR/Cas9 technology.
- Characterize the specific phenotype of MED12-knockout and MED12-mutated cell lines.
- Analyze the sensitivity of our generated models to conventional HNSCC treatment with paclitaxel, 5-fluorouracil and cisplatin.
- Validate and experimental design that allows the MED12 silencing in HNSCC cells.

Particularly, the inventors of the present invention have investigated the role of MED 12 in HNSCC and explores its potential as a therapeutic target. Using CRISPR/Cas9 technology, three distinct MED12-edited HNSCC clones were created, targeting the N-terminal region, specifically exon 2. Sanger sequencing confirmed homozygotic deletions in the N-terminal region. Protein expression analysis revealed that one clone exhibited no protein expression, while the other two continued to express MED 12 protein, highlighting the complexity and variability of gene editing outcomes. Functional characterization of these models revealed a significant reduction in cell proliferation, indicating the critical role of the N-terminal domain in regulating this biological process. Moreover, disruption of MED12 protein expression promoted cell migration. Thus, targeting only the N-terminal domain and not the whole protein might be particularly effective, as it inhibits proliferation while maintaining low migration rates and the response to conventional chemotherapy. Additionally, shRNA silencing emerged as a quicker approach to gene inactivation compared to CRISPR/Cas9, achieving similar results.

So, the present invention highlights the oncogenic vulnerability of MED12, particularly within its N-terminal domain, underscoring its potential as a therapeutic target in HNSCC.

According to the Examples provided below, the following conclusions were reached:
- CRISPR/Cas9 technology can produce diverse phenotypes depending on whether it results in a complete gene knockout or the expression of aberrant proteins.
- The loss of MED 12 inhibits cell proliferation and promotes cell migration in HNSCC.
- Targeting the MED12 N-terminal is a potential therapeutic strategy in HNSCC as its alteration inhibits cell proliferation without altering migration rates nor the response to conventional chemotherapy.
- MED12 shRNA silencing constitutes a quicker and less aggressive approach for gene inactivation compared to CRISPR/Cas9, achieving similar results.

Although the present invention refers to the treatment of HNSCC as proof-of-concept, other solid tumours could be treated with this strategy, for instance ovarian cancer.

So, the first embodiment of the present invention refers to MED12 inhibitors, or pharmaceutical composition comprising thereof, for use in a method for the treatment of cancer. Alternatively, the first embodiment refers to a method for treating cancer which comprises the administration of a therapeutically effective dose or amount of MED 12 inhibitors, or a pharmaceutical composition comprising thereof.

The second embodiment of the present invention refers to a combination drug product comprising a MED12 inhibitor and chemotherapy.

The third embodiment of the present invention refers to a pharmaceutical composition comprising the combination drug product of the invention and, optionally, pharmaceutically acceptable excipients or carriers.

The fourth embodiment of the present invention refers to an *in vitro* method for screening, identifying and/or producing compounds for use in the treatment of cancer which comprises: a) Assessing MED12 gene expression once the candidate compound has been incubated with MED12 gene, and b) wherein if an inhibition of MED12 gene expression is observed, it is indicative that the candidate compound may be effective for use in the treatment of cancer.

In a preferred embodiment, the inhibitor is a genetic inhibitor designed to interfere with the expression or function of MED12 gene.

In a preferred embodiment, the inhibitor is shRNA, siRNA, CRISPR-Cas9 or anti-MED12 antibodies.

In a preferred embodiment, the inhibitor is a small molecule inhibitor.

In a preferred embodiment, the MED12 inhibitor is administered in combination with chemotherapy (i.e., MED12 inhibitor is administered after, before or concomitantly to the chemotherapy).

In a preferred embodiment, the chemotherapy used in the context of the present invention pertains to the common general knowledge, for instance the chemotherapy disclosed in this review [Uttpal Anand, Abhijit Dey, Arvind K. Singh Chandel, Rupa Sanyal, Amarnath Mishra, Devendra Kumar Pandey, Valentina De Falco, Arun Upadhyay, Ramesh Kandimalla, Anupama Chaudhary, Jaspreet Kaur Dhanjal, Saikat Dewanjee, Jayalakshmi Vallamkondu, José M. Pérez de la Lastra, Cancer chemotherapy and beyond: Current status, drug candidates, associated risks and progress in targeted therapeutics, Genes & Diseases, Volume 10, Issue 4, 2023, Pages 1367-1401, ISSN 2352-3042, https://doi.org/10.1016/j.gendis.2022.02.007], preferably taxanes (paclitaxel/docetaxel), cisplatin and/or 5-fluorouracil (5-FU)

In a preferred embodiment, the cancer is a solid tumour, for instance head and neck or ovarian cancer.

For the purpose of the present invention the following terms are defined:
- The term "comprising" means including, but not limited to, whatever follows the word "comprising". Thus, use of the term "comprising" indicates that the listed elements are required or mandatory, but that other elements are optional and may or may not be present.
- The term "consisting of" means including, and limited to, whatever follows the phrase "consisting of'. Thus, the phrase "consisting of" indicates that the listed elements are required or mandatory, and that no other elements may be present.
- The expression "therapeutically effective dose or amount" refer to an amount that, when administered as described herein, brings about a positive therapeutic response in a subject having cancer. The exact amount required will vary from subject to subject, depending on age, and general condition of the subject, the severity of the condition being treated, mode of administration, and the like. An appropriate "effective" amount in any individual case may be determined by one of ordinary skill in the art using routine experimentation, based upon the information provided herein.

### Description of the figures

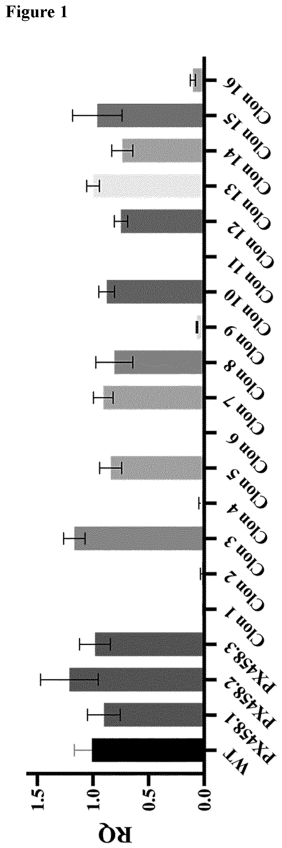

**Figure 1****.** Normalization of the amplified DNA for the fragment of interest in the MED12 gene by quantitative real-time PCR. The WT cell line is used as reference. Three control clones (empty PX458) and sixteen clones transfected with the G1+G2 combination are represented.

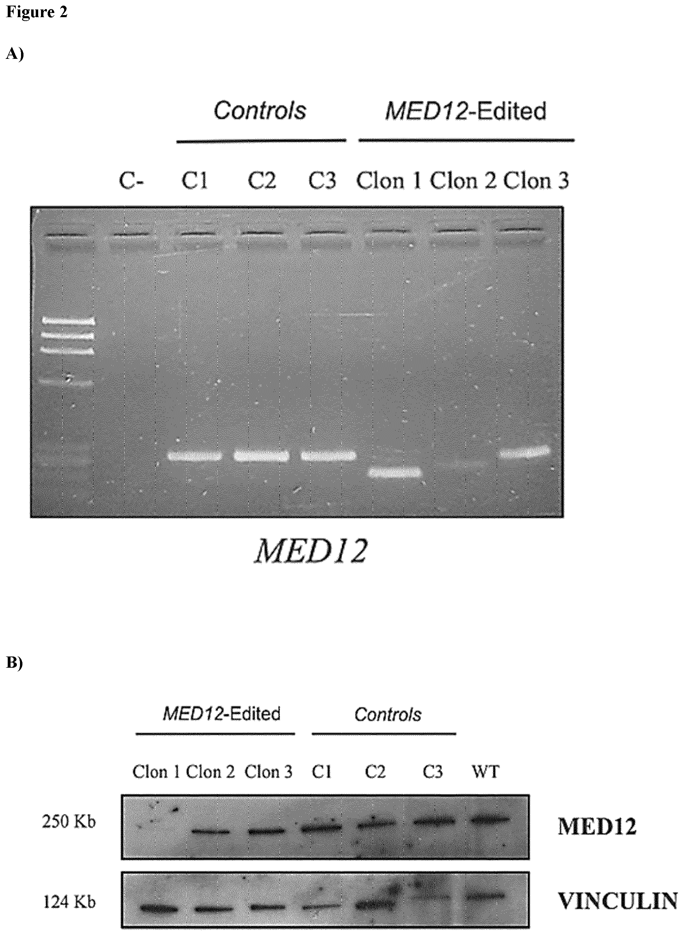

**Figure 2****.** Confirmation of the successful CRISPR/Cas9-mediated generation of MED 12-edited models. **A)** Agarose gel electrophoresis of PCR products with primers specific for the MED12 gene. **B)** Protein expression analysis of MED12 in 32816 WT, three control clones and three CRISPR/Cas9-edited clones.

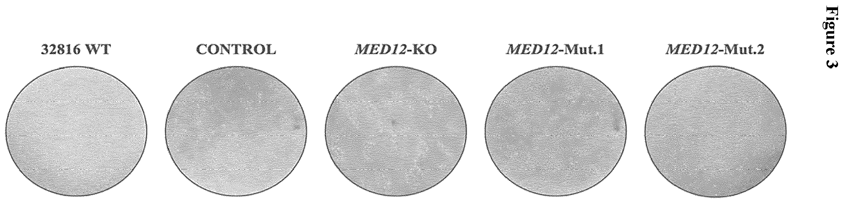

**Figure 3****.** Microscopy Images (X20) of cell growth from the 32816 WT alongside the different generated clones.

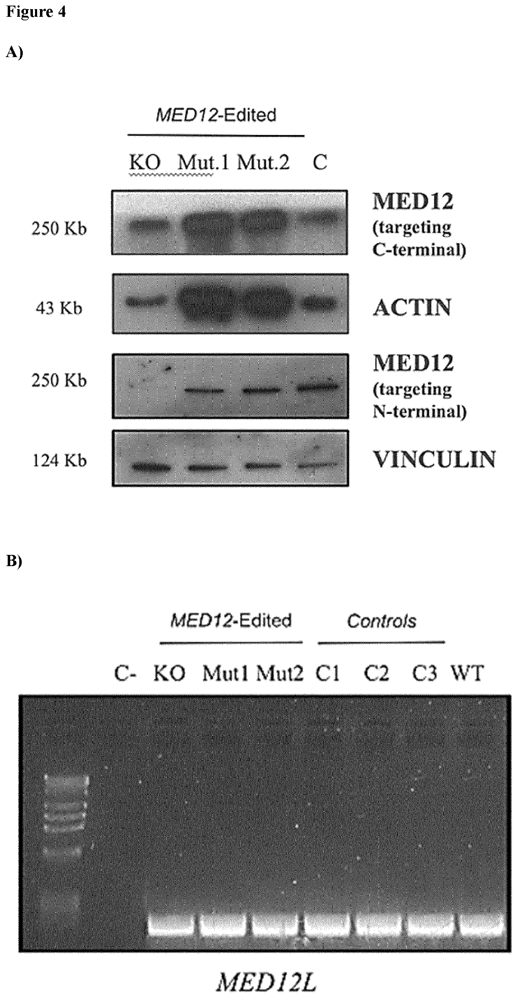

**Figure 4****.** Validation of two anti-MED12 commercial antibodies. The figure indicates a cross-reaction between MED 12 and MED12-Like in our clones when using a C-terminal targeting antibody. **A)** MED12 protein levels of expression of the three edited clones compared to control and WT cells by western blot. **B)** Confirmation of MED12-Like presence in our clones by cDNAPCR.

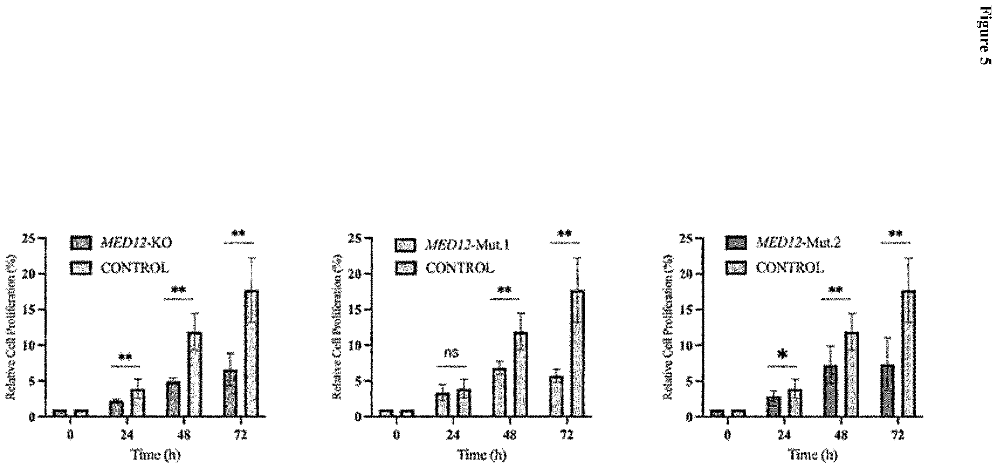

**Figure 5****.** MTT assay results on cell proliferation. The figure displays the relative cell proliferation rate along with the correspondent standard deviation at 4 time points (0,24, 48 and 72 hours) of the three MED12-edited clones independently compared to the average of the three control clones. *p-value < 0.05; **p-value < 0.001.

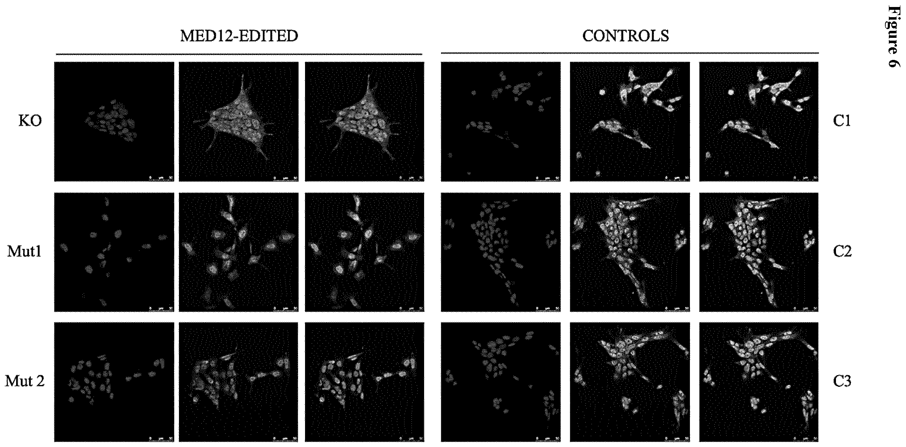

**Figure 6****.** Inmunofluorescence confocal microscopy images. The figure shows MED12-edited and control cells stained with DAPI (blue) to visualize nuclei and anti-CDK8 (green). Sacale bar: 25 µM.

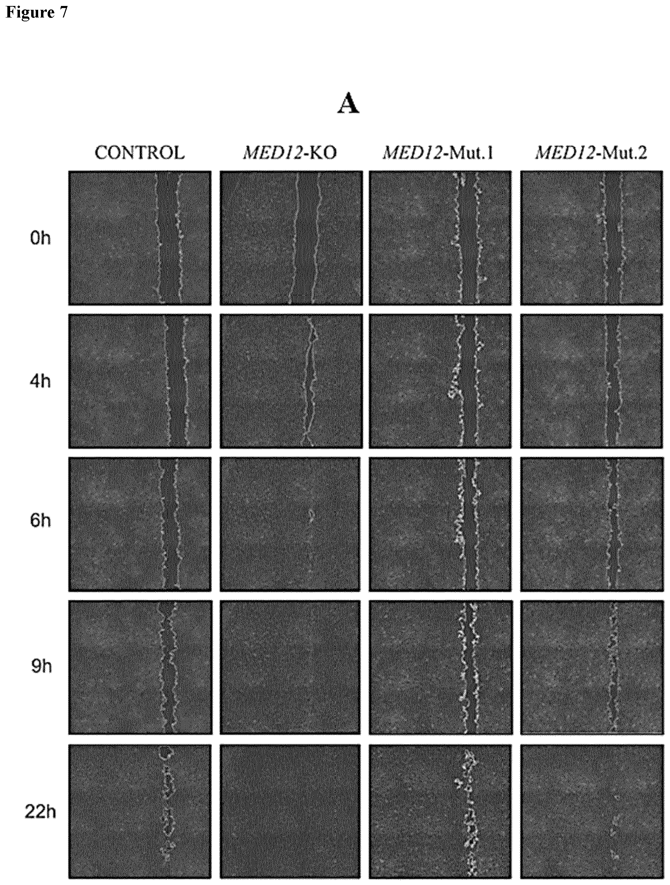

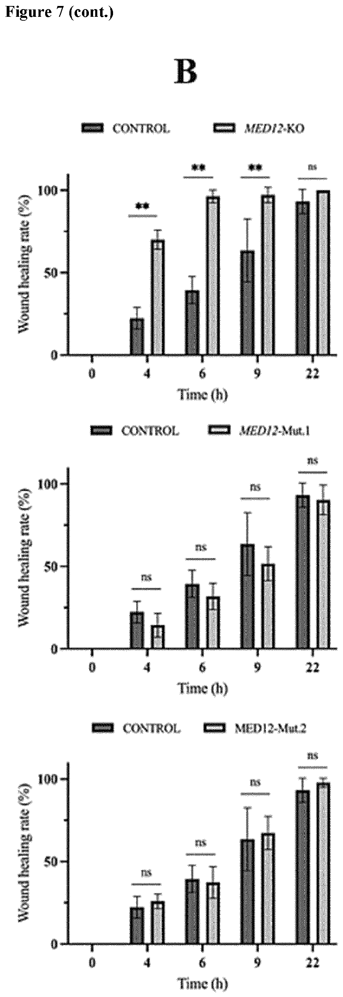

**Figure 7****.** Analysis of Migration Assay. **A)** Photos taken at 0, 4, 6, 9 and 22 h of the "wound healing" assay. The figure displays one of the four replicates for the three MED12 conditions and one control clones. It is noted that at 6 hours, the wounds in the MED12 knockout clone are the only ones completely closed. **B)** Representation of the migration percentage for each of the 2 conditions compared with the average of the three controls at the 5 time-points

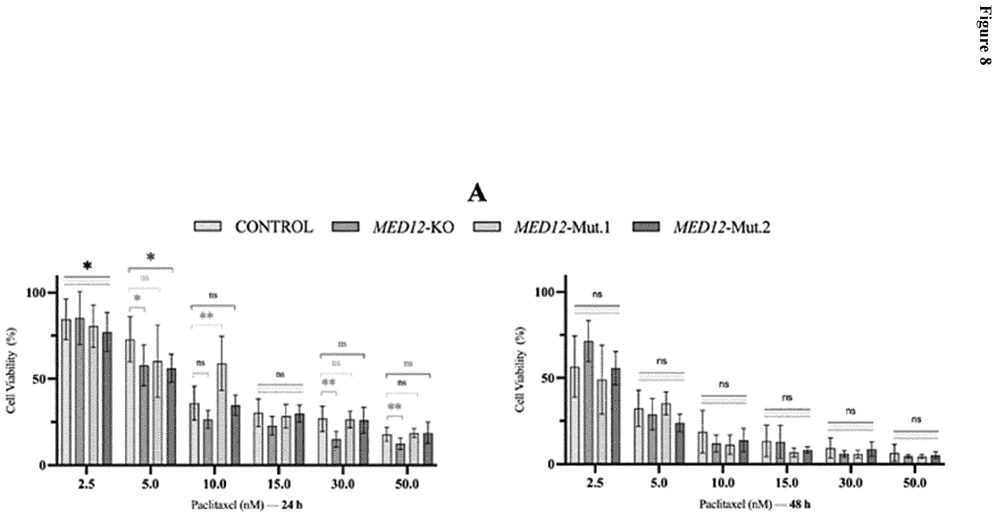

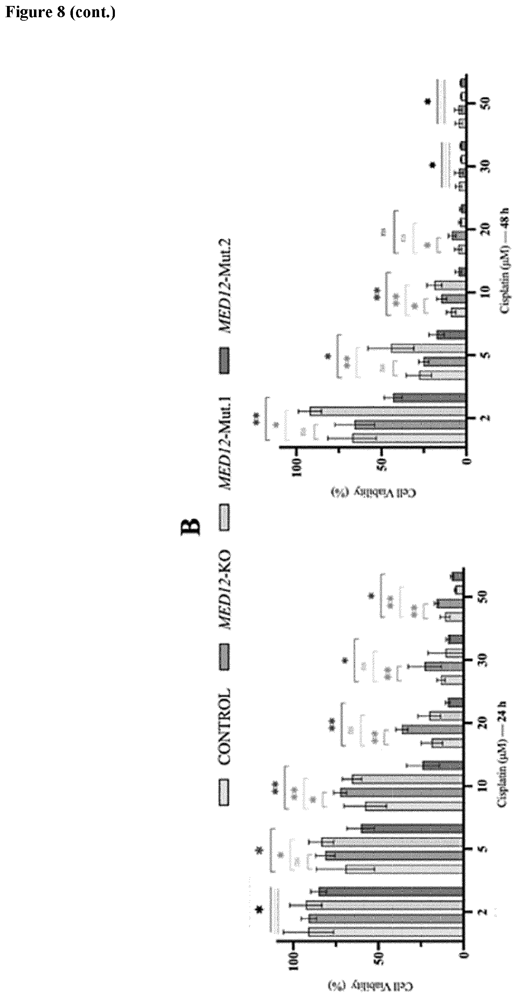

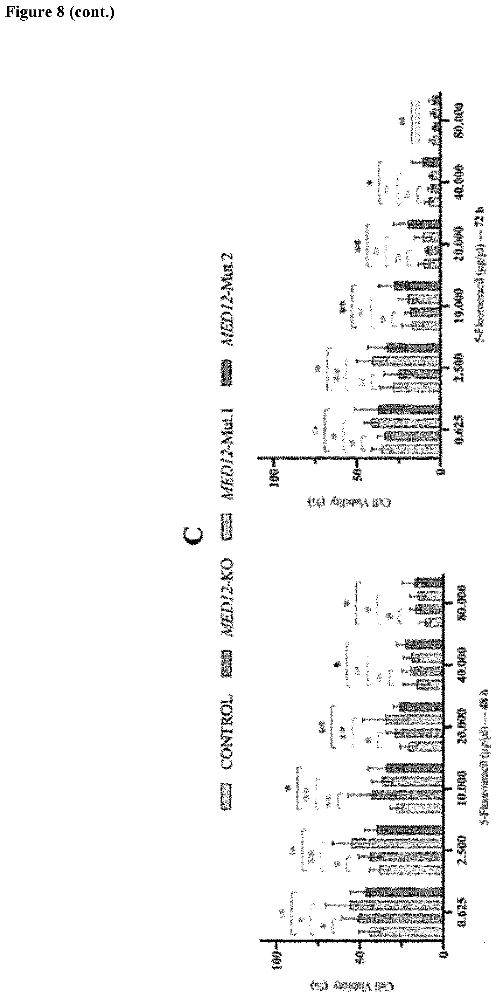

**Figure 8****.** MTT assay results on response to drugs included in the TPF chemotherapy regimen. The figure displays the average viability percentage along with the corresponding standard deviation for each MED 12 condition and the average of three control clones at 6 concentrations and two time points). *p-value < 0.05; **p-value < 0.001. A) Response to paclitaxel (B) Response to cisplatin C) Response to 5-fluorouracil (5-FU.)

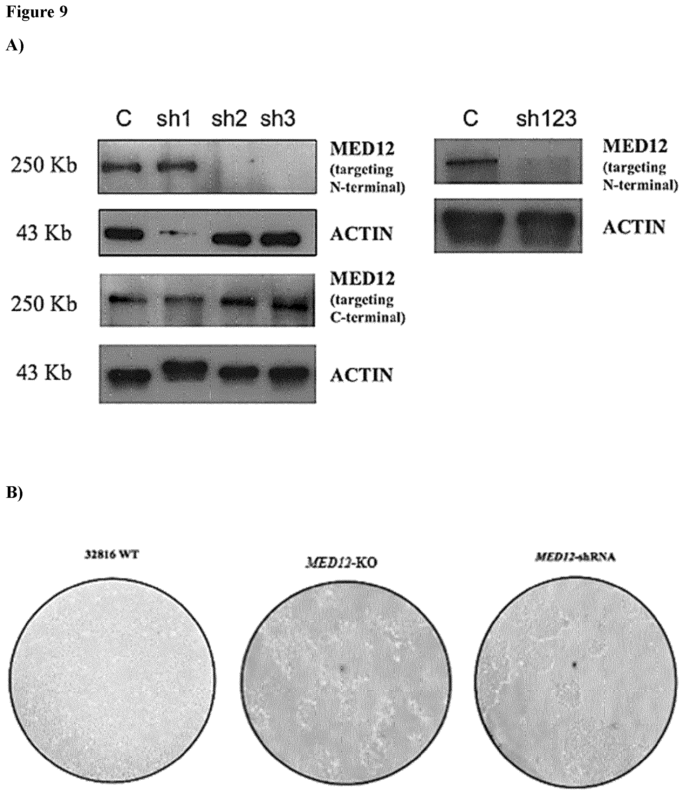

**Figure 9****.** Preliminary characterization of MED12-silenced cells. **A)** Validation of protein expression silencing of the different shRNAs by western blotting. **B)** Microscopy Images (X20) of cell growth from the 32816 WT, the MED12-KO clones and one of the MED12-silenced population (sh2).

### Detailed description of the invention

The present invention is illustrated by means of the Examples set below, without the intention of limiting its scope of protection.

### Example 1. Material and methods

### Example 1.1. Cell Culture

The 32816-cell line used in the study was previously generated in our laboratory from an oropharynx squamous cell carcinoma biopsy of a 73-year-old male obtained from the Hospital of Salamanca. Its main characteristics are summarized in **Table 1.**

**Table 1. Main characteristics of the cell line included in the study.**

| Name | Origin | Tumor Type | VPH status |
|---|---|---|---|
| 32816 | Tumor biopsy of a 73-year-old male patient, smoker. University Hospital of Salamanca | Oropharyngeal squamous cell carcinoma | + |

The cell line grew in Roswell Park Memorial Institute 1640 (RPMI) medium (Life Technologies, Paisley, UK) supplemented with 10% fetal bovine serum (FBS) and 1% penicillin- streptomycin (Life Technologies, Grand Island, NY, USA). The cells were cultured in an adherent monolayer in 10 cm diameter culture plates (BD FalconTM) at 37°c and 5% CO2 and 98% relative humidity. Weekly passages were performed when cell confluence reached 80% to allow appropriate cellular growth and the presence of mycoplasma was routinely checked with the MycoAlert kit (Lonza, Basel, Switzerland). Only mycoplasma-free cells were used in all experiments included in this work.

### Example 1.1.1 Defrosting

The 32816-cell line was frozen at -196°C in a cryovial containing 1 ml of freezing medium made of fetal bovine serum (FBS) (Life Technologies, Paisley, UK) with 10% dimethyl sulfoxide (DMSO) (Sigma, St Louis, MO, USA). The vial was defrosted in a 37°C water bath. And added to a properly labeled 10 cm diameter culture plate containing 11 ml of supplemented RPMI culture medium. The next day, the plate was washed twice with phosphate-buffered saline (PBS) and 10 ml of new medium was added to avoid the toxic effects of DMSO.

### Example 1.1.2. Cell Counting

Cell counting was performed with a Neubauer chamber for all experiments requiring a specific number of seeded cells. From each cell line, 30 µL of previously trypsinized cells resuspended in 6 mL of RPMI medium were added to the chamber. Subsequently, cells in one of the cambers were counted by calculating the average of 4 different quadrantes under an optical microscope. Thus, considering the dilution factor of the chamber (1:10000), the cell concentration of the sample was calculated as follows: (Total number of observed cells / 4 quadrant) x 1000 = Number of cells / mL.

### Example 1.2. Gene Editing by CRISPR/Cas9 Technology

CRISPR/Cas9 gene editing is based on two main components: the guide RNA (gRNA) and the Cas9 protein. The gRNA is designed to bind the target DNA sequence, guiding the Cas9 protein to the precise location in the genome. Cas9 then acts as molecular scissors, creating a double-strand break in the DNA. The natural repair mechanisms of the cells can lead to either random insertions or deletions via non-homologous end joining (NHEJ), which typically disables the gene, or the introduction of new genetic material through homologous recombination repair (HRR), allowing for precise modifications.

### Example 1.2.1. sgRNAs Design

Two sgRNA guides were designed to target the MED12 gene in the 32816-cell line with the wild type (WT) gene. Exon 2, out of the 46 exons in this gene, was selected as the target sequence. This choice was based on two premises:
1. The region is located at the beginning of the gene sequence, being more likely to generate a premature stop codon after the Cas9 cut site, resulting in a truncated protein.
2. Exon 2 is proven to be critical for the proper functioning of MED 12 and constitutes a hotspot of alterations linked with numerous diseases, including cancer.

The sgRNAs design was carried out using the CRISPR-Cas9 guide RNA design checker online tool (https://eu.idtdna.com/site/order/designtool/index/CRISPR_SEQUENCE), developed by Integrated DNA Technologies (IDT). The sequence of the fragment to be edited was inserted in FASTA format and the tool provides candidate sgRNAs along with the oligonucleotides needed to construct them and a score regarding efficiency and specificity regarding on-target and potential off-target cuts. Two sgRNA with scores near 100%, specified in **Table 2,** were selected to edit the MED 12 gene.

**Table 2. Target sequences and oligonucleotides for the construction of MED12 sgRNAs.**

| | Target sequence | Oligo-High | Oligo-High |
|---|---|---|---|
| sgRN A-1 | ACGGCCTTGAATGTAA AACA (SEQ ID NO: 1) | 5'caccgACGGCCTTGAATGTA AAACA (SEQ ID NO: 3) | 5′aaacTGTTTTACACTTCAAG GCCGTc (SEQ ID NO: 5) |
| sgRN A-2 | ATAACCAGCCTGCTGT CTCT (SEQ ID NO: 2) | 5'caccgATAACCAGCCTGCTG TCTCT (SEQ ID NO: 4) | 5′aaacAGAGACAGCAGGCYG GTTATc (SEQ ID NO: 6) |

### Example 1.2.2. Plasmid construction

The pSpCas9(BB)-2A-GFP (PX458, ref: #48138) plasmid was used as a vector for the CRISPR/Cas9 system. The plasmid contained the sequence for the Cas9 endonuclease and the green fluorescent protein EGFP, which was later used for selecting cells that incorporated the plasmid. Both sequences are under the control of the CMV promoter. However, the sgRNAs needed to be inserted into the plasmid to complete the construction. The insertion of both designed sgRNAs was performed independently through 3 consecutive steps.

First, each pair of oligonucleotides was phosphorylated and annealed using T4 polynucleotide kinase (T4 PNK), which catalyzes the transfer of the phosphate group to the oligos. The resulting product was diluted 1:250. Next, the digestion-ligation reaction was performed using the enzyme BpiI, whose restriction site is found in the PX458 plasmid. The product was then treated with PlasmidSafe exonuclease to prevent the formation of undesired recombination products.

After modifying the vector, competent DH5-α Escherichia coli bacteria were transformed. 2 µL of the vector with the sgRNA 1 inserted were added to 50 µL of bacteria. The same step was repeated first with the vector containing sgRNA1 and second with an empty vector as a control. The Eppendorf with the mix was incubated on ice 30 minutes after which it undertook heat shock of 42°C for 30 seconds followed by 5 minutes on ice. Afterwards, 250 µL of prewarmed 2XTY medium without ampicillin was added to promote bacterial growth, and the tubes were maintained at 37°C with shaking for 1 hour. Then, they were centrifugated at 14.000 rpm for 10 seconds to pellet the bacteria, which was subsequent resuspended in 50 µL of 2XTY medium and spread onto the plates using a Drigalski spatula. The plates were incubated overnight at 37°C. The ampicillin resistance gene of the plasmid should ensure that growing colonies have incorporated the vector.

Next day, isolated colonies were picked with sterile toothpicks and transferred to 1.5 mL tubes containing 1 mL of 2XTY medium. These were again incubated overnight at 37°C to promote growth of bacterial cultures and allow the extraction of plasmid DNA from them. The extraction and subsequent purification of DNA was achieved by performing minipreps using the Danagene plasmid miniprep kit (DANAGEN-BIOTED S.L., Barcelona, Spain; ref. 0701), following the manufacturer's specifications.

Sanger sequencing was performed at the Genomics Unit of the Cancer Research Center of Salamanca (CIC) to confirm the correct incorporation of the guides into the PX458 plasmid. The sequences were analyzed with Snapgene Viewer Software to confirm the guide insertion, and therefore, the correct plasmid construction. Once this was confirmed, colonies were picked again and cultured overnight in flasks with 200 mL of 2XTY medium with ampicillin. Maxipreps were then performed to obtain a larger quantity of plasmid DNA using The Thermo Fisher Scientific kit (ref. K210007 with steps of lysis, neutralization, DNAbinding to a column, washing and elution. Final DNA concentration was measured with NanoDrop^{®}.

### Example 1.2.3. Cell Line Electroporation

The generated plasmids were transfected into the 32816-cell line by electroporation. 1 million cells per condition were resuspended in 100 µL of electroporation medium (150 µL of 4 MgCL2 every 10 mL of succinate-manitol) and placed in an Eppendorf. For the first condition, 2.5 ug of plasmid containing sgRNA1 and 2.5 ug of sgRNA-2 plasmid were added to transfect both sgRNAs combined enhancing the efficiency. For the second condition, 5 ug of the empty plasmid were added as a control. Each condition was transferred to an electroporation cuvette and cells were electroporated using the T-020 program of Amaxa Nucleoefector device. Afterwards, cells were collected with a Pasteur pipette and added to wells of a 6-well plate containing 2 mL of supplemented RPMI medium.

### Example 1.2.4. Cell sorting through flow cytometry

48 hours after electroporation, cells that had incorporated the construct of interest were isolated into 96-well plates by flow cytometry. Specifically, separation was based on the green light emission of the plasmid EGFP gene, detected by BD FACSArialTM III flow cytometer. Each single cell was left to grow for 3 weeks in supplemented RPMI medium with 20% of FBS to generate clones containing the sgRNA1 and 2 as well as control clones with the empty plasmid. However, the incorporation of the plasmid does not guarantee the desired MED 12 gene editing nor its inactivation. Therefore, once the clones were generated, they were expanded through consecutive passages from the 96-well plate to 48, 24, 6-well plates and 10 cm petri dishes. At this point, DNA, RNA and protein extractions from the different clones were performed to confirm the CRISPR/Cas9-mediated gene editing of MED12.

### Example 1.3. Gene Silencing by shRNAs

The human MED12 shRNA plasmid (Abbexa; Ref: abx956705) was used for the silencing of the MED12 gen in our cell line. The plasmid contains the EGFP gene, a neomycin-resistant gene for selection and three distinct shRNA sequences detailed as follows:
MED12-sh1: 5'-GGTTAAGAAGAGACATGTTGA-3' (SEQ ID NO: 7),
MED12-sh2: 5'-GCTGGCCATGTTCATGTTTCA-3' (SEQ ID NO: 8), and
MED12-sh3: 5'-GC TGTTCTGTGAACTGATTCG-3'(SEQ ID NO: 9).

Each sequence was transfected individually and in combination into the 32816-cell line using the same electroporation method previously described for the CRISPR/Cas9 system. The empty pCDNA plasmid (Invitrogen; Ref: V79020), which includes the same neomycin resistance gene but lacks the shRNA sequences, was also transfected as a control. Electroporation efficiency was assessed 48 hours post-transfection by detecting the green fluorescence emitted from the plasmid's EGFP gene using a BD FACSArial^{™} III flow cytometer. At this time point, selection of cells that had incorporated the plasmid initiated, facilitated by the neomycin resistance gene included in the plasmid. Cells cultured in 6-well plates were treated with 600 µg/mL of neomycin (G418) every three days for seven days. A wild-type control well was included to ensure complete death of non-transfected wild-type cells within this period. Following selection, the cells were expanded into a Petri dish, and protein extraction was performed to evaluate the silencing efficiency of each transfection (sh1, sh2, sh3, and sh1-2-3) relative to the control.

### Example 1.4. Nucleic Acid Extraction from Cell Lines

Cells of the generated clones were collected using 1 mL of 1X TrypLE dissociation agent (GibcoTM), washed with 2 mL of PBS, and centrifuged at 1200 rpm for 5 minutes.

### Example 1.4.1. DNA extraction

For DNA extraction, cell pellet was resuspended in 1 mL Fornace buffer with 10 mM EDTA to promote nuclease inactivation, 50ug/mL proteinase K (ApliChem) to degrade proteins, and 1% SDS to disrupt the cell membrane. Following a 24 hours-incubation at 55°C, DNA extraction and purification were performed by treating the sample with 1 volume of phenol and centrifugating it at 1800 rpm for 10 minutes. The upper aqueous phase containing the DNA was mixed with one volume of CIAA (chloroform: isoamyl alcohol 24: 1) to eliminate cellular residues and after a second centrifugation, the aqueous phase was again collected. Cold absolute ethanol was added to precipitate the DNA and a final wash with 70% ethanol was conducted. DNA was resuspended in 200 µL of ddH2O.

### Example 1.4.2. RNA extraction

For RNA extraction, cells were resuspended in 1 mL of Trizol (Life Technologies) and transferred to and Eppendorf. 200 µL of chloroform was added, vortexed and left at room temperature for 10 minutes. Centrifugation at 12000 g for 15 minutes (4°C) was then conducted to separate the upper aqueous phase containing RNA that was transferred to another tube. 500 µL of cold isopropanol were added to precipitate the RNA for 24 hours at -80°C. Then, the sample was centrifuged at 12000 g (4°C) for 10 minutes. The supernatant was eliminated, leaving the RNA, which was washed with 1 mL of 70% cold ethanol and resuspended in 40 µL of RNase-free water. The concentration of nucleic acids and was determined using a NanoDrop^{®} 2000 spectrophotometer (Thermo Fisher Scientific) by measuring the absorbance of the sample at 260 nm. The ratio of absorbances A260/A280 indicated the sample purity with a correct range of 1.8-2.0. Only good quality samples were used for subsequent experiments, and to prevent their degradation they were stored at -20°C (DNA) and -80°C (RNA).

### Example 1.4.3. Complementary DNA (cDNA) synthesis via reverse transcription (RT)

0.5-2 ug of final extracted RNA was used to synthesize cDNA to quantify gene expression at the transcriptomic level. To do this, the High-Capacit-cDNA-Reverse-Transcription-Kit (BiosystemsTM) was used. 2 µL of 10X RT Buffer, 0.8 µL of 25X dNTP Mix (100 mM), 2 µL of 10X RT Random Primers, 1 µL of MultiScribeTM Reverse Transcriptase, 1 µL of RNase Inhibitor, and 3.2 µL of H2O was added to the RNA sample. Then, for the synthesis reaction the following program was run in a thermocycler: 10 min 25°C - 120 min 37°C - 5min 85°C - 4°C. The obtained cDNA was stored at -20°C.

### Example 1.5. Protein Extraction from cell lines

Protein extraction from the generated clones was consistently performed on ice to maintain sample integrity. Each plate, pre-washed twice with PBS, received 0.6 mL of lysis buffer composed of 45.5 mL ddH2O, 2.5 mL 1M Tris, 1.5 mL 5MNaCl, 100 µL 0.5M EDTA, and 0.5 mL Triton. Following a 1-minute incubation period, the plate was scraped to collect the buffer containing the extracted proteins, which were then transferred to new tubes kept on ice. Throughout the 45-minute incubation, tubes were vortexed every 10 minutes to ensure thorough mixing. Subsequently, the tubes underwent centrifugation at 1400 rpm (4°C) for 15 minutes. Finally, the supernatant was transferred to properly labelled Eppendorf tubes. Protein quantification was then performed by using the Bradford method, based on the "Coomassie brilliant blue G-250" dye that binds to the proteins in the sample changing the color from brown to blue indicating a shift in its absorption maximum (from 465 nm to 595 nm). Independent tubes were prepared with increasing and known concentrations of bovine serum albumin (BSA) to determine a standard curve and the different protein extracts. After mixing the tubes with 20 µL of diluted protein extract in ddH20 and 980 µL of Bradford reagent and incubating in dark 30 minutes, the samples were measured for triplicate at 595 nm using the TECAN spectrophotometer and concentrations were calculated according to the standard curve obtained.

### Example 1.6. Polymerase Chain Reaction (PCR) and Sanger Sequencing

Amplification of MED12 gene from 50 ng of DNA and cDNA, independently, by PCR was conducted in a thermocycler (Life Technologies-Invitrogen) to study the CRISPR/Cas9 genomic and transcriptomic changes in the edited clones, specifically within the exon 2 region. For confirming the presence of the MED12L gene expression in the 32816-cell line, only cDNA samples were used. The oligonucleotides and the amplification program are indicated in **Table 3.** All PCR products were loaded onto a 0.5X Tris/Borate/EDTA (TBE) buffer gel with 2% agarose to confirm the amplification of the region. The gel was run for 25 minutes at 120V.

**Table 3. Oligonucleotides used for the amplification of MED12 and MED12L gene by PCR.**

| Gene | Forward Primer (5'→ 3') | Reverse Primer (5'→ 3') | Amplicon Size |
|---|---|---|---|
| *MED12* (DNA; exon 2) | TCCTAGTGACCATGGGAGTGA (SEQ ID NO: 10) | GGTCATGAAGGCAAACTCAGC (SEQ ID NO: 11) | 412 pb |
| *MED12* (cDNA; exon 1-3) | CGGGATCTTGAGCTACGAACA (SEQ ID NO: 12) | TCCTTCTGGTTCACTTGGGG (SEQ ID NO: 13) | 299 pb |
| *MED12L* (cDNA; exon 3-4) | AGCCAGCCTTCACTGGAGAT (SEQ ID NO: 14) | CTGGGATCGAGCAGTAACCA (SEQ ID NO: 15) | 188 pb |

The product of the MED 12 PCR from DNA and cDNA fragments was then purified using a kit from Life Technologies (ref. K310002) for sequencing using the sange method to determine the specific alterations caused after the Cas9 action. Thus, the purified products were sent to the Genomics Unit of the Cancer Research Center of Salamanca (CIC) in a total volume of 8 µL with 2 µL of forward primer. The sequences were analyzed using the SnapGene Viewer Software.

### Example 1.7. Quantitative PCR (qPCR)

Quantitative PCR (qPCR) was performed to confirm the inactivation of the MED12 N-terminal of the clones at the transcriptomic level. For this, 2 µL of cDNA at a 20 ng/µL concentration were mixed with 5 µL of SYBR Green Master Mix, 1 µL of each specific primer (forward and reverse) and 1 µL of ddH20 for a total volume of 10 µL. A StepOnePlusTM Real-Time PCR System, (Life Technologies-Invitrogen) was used to perform the reaction.

All reactions were performed per quadruplicate and GAPDH gene was amplified as internal control. The sgRNAs designed guides, detailed in **Table 2,** were used as primers used for the amplification of the specific MED 12 region of interest from cDNA samples of the edited and control clones.

### Example 1.8. Western Blot

After confirming the genetic editing of MED12 in both DNA and RNA transcripts, a Western blot analysis was conducted to examine the impact of these alterations on protein expression and to determine the potential inactivation of the MED12 protein.

### Example 1.8.1. SDS-PAGE electrophoresis

Extracts with the same total protein amount (20 ug) were prepared, to which loading buffer (100 mM Tris pH 6.8, 20% glycerol, 4% SDS, 0.05% bromophenol blue, and 2% β-mercaptoethanol) was added. The extracts were denatured at 95°C for 5 minutes in a dry bath and loaded onto an 8% polyacrylamide gel that was previously prepared in two consecutive steps. First, the resolving gel was prepared to separate the proteins by size. The specific composition includes Tris buffer (1.5 M Tris-HCl, ph 8.8 and 10% SDS), acylamide and ddH20. On top of it, the stacking gel was prepared by mixing Tris-buffer (0.125 M Tris-HCl, ph 6.8 and 3.5 mM SDS), acrylamide and water. For the polymerization of both gels, ammonium persulfate (APS) and tetramethyl-ethyl-enediamine (TEMED) were added. The electrophoresis was conducted at a constant voltage of 80 volts the first 10 minutes and 140 volts for approximately 90 more minutes. The PageRuler^{™} Prestained Protein Ladder Plus (Thermo Fisher Scientific) was used as the molecular weight marker.

### Example 1.8.2. Semi-wet transfer and Incubation with Antibodies

Once electrophoresis finished, the proteins were transferred from the gel to a PVDF Immobilon-P membrane (Millipore Corporation) with the semi-dry transfer system TRANS BLOT ^{®} (Bio-Rad) for 25 minutes. Transfer buffer containing 25 mM Tris-HCl, 192 mM glycine and 20% methanol was used. Following the transfer, the membrane was blocked for 1 hour shaking at room temperature in 10 mL of Tris-buffered saline with 0.1 % of tween 20 (TBST-T) containing 5% of dry milk. Then, the membrane was incubated overnight at 4°C with the required primary antibody (**Table 4**).

**Table 4. List of antibodies used for western blot in this study.**

| Antibody I | House | Dilution | Host | Antibody II | Dilution |
|---|---|---|---|---|---|
| *Anti-MED12* | ABCAM, AB70842 | 1:2000 (3% BSA in TBST) | Rabbit | Anti-Rb (Goat) | 1:10000 (5% milk in TBST) |
| *Anti-MED12* | TMO, PA5-100279 | 1:1000 (3% BSA in TBST) | Rabbit | Anti-Rb (Goat) | 1:10000 (5% milk in TBST) |

After incubation, the membrane was washed 3 times for 5, 7 and 10 minutes respectively with TBS-T and with shaking, followed by incubation with the secondary antibody (**Table 4**) with shaking for one hour at room temperature. Then, the previous 3 washes were conducted again.

### Example 1.8.3. Protein Detection and Visualization

Lastly, the protein's chemiluminescent detection was conducted by adding to the membrane 1 µL of H202 andt1 mL of the ECL revelation reagent which binds to the peroxidase of the secondary antibody, emitting light detectable when exposing the membrane to X-ray film (Fujifilm medical X-ray). The peroxidase catalyzes the oxidation of luminol in the presence of hydrogen peroxide. The exposure time varies depending on the primary antibody used.

### Example 1.9. Immunofluorescence

Inmunofluorescence assays were conducted to determine the localization of CDK8 protein when MED12 protein expression was aberrant or disrupted. To carry out the assay, 300,000 cells from each condition were seeded into individual wells of a 6-well plate, each containing three coverslips.

### Example 1.9.1. Cell Fixation and Incubation with Antibodies

After 24 hours of incubation at 37°C with 5% CO2 cells that were attached to the coverslips were fixed with 4% formaldeyde in PBS for 10 minutes. Next, cell permeabilization was done by adding 2 mL of Triton diluted in PBS (0,2%) and incubating on ice 10 more minutes. To prevent nonspecific antibody binding, cells were incubated for 90 minutes with 2 ml of a 10% BSA solution in PBS. Subsequently, cells undertook 3 5-minute washes with 2 mL of PBS. After that, 90 minute-incubation with 30 µL of the primary antibody (**Table 5**) was performed in the coverslips previously transferred from the 6-well plate to a wet chamber (Petri dish with double wet filter paper and parafilm as a cover).

**Table 5. List of antibodies used for immunofluorescence in this study.**

| Antibody I | House | Dilution | Host | Antibody II | Concentration |
|---|---|---|---|---|---|
| *Anti-CDK8* | STA.CRUZ, SC-13155 | 1:200 (3% BSA in TBST) | Mouse | Anti-Ms (ALEXA FLUOR 488) | 1:1000 (1%BSA in TBST) |

Following three 5-minute washes with 100 µL of PBS, incubation of the secondary antibody for one hour took place in the dark since it has fluorescence associated (**Table 5**). Again, three 5-minute washes were performed to finaly stain the cell nuclei with 100 µL DAPI at 1:2000 dilution in ddH20 from a 1 ug/µL stock solution. Subsequently, cells were wash 3 times again.

### Example 1.9.2. Mounting and Image Capture

The final step was to mount the coverslips on labelled slides with 15 µL of Mowiol^{®} (Sigma-Aldrich, Germany, ref. 81381). Three coverslips with the cells facing downwards were placed onto each slide and were left to dry in darkness. The slides were stored at 4°C until fluorescence imaging with a LEICA TCS SP5 DMI-600B confocal microscope (Leica Microsystems) (63X).

### Example 1.10. Cell viability MTT assay

The compound MTT, 3-(4,5-dimethylthiazol-2-yl)-2,5-diphenyltetrazolium bromide (MTT) (Sigma-Aldrich), is a yellow tetrazolium salt that is reduced to purple formazan in metabolically active cells by the action of the enzyme succinate dehydrogenase (SDH). The formazan is solubilized with dimethyl sulfoxide (DMSO), allowing the absorbance of the samples to be measured with a spectrophotometer. This absorbance directly correlates with metabolically active cells.

### Example 1.10.1. In vitro cytotoxicity assay

Cytotoxicity of conventional drugs included in the HNSCC regimen was tested for the MED12-edited clones compared to the control ones by MTT assay. 2000 cells/well from each clone were seeded in a 96-well plate and cell viability was measured at two time-points (48-72h for 5-FU, 24-48h for cisplatin and paclitaxel) after treatment with different concentrations (**Table 6**) administrated in quadruplicate. A blank measure (without cells) and a control one (without drug) was included in all cases.

**Table 6. Conventional HNSCC drugs included in this work.**

| Drug | Commercial House | Stock | Doses |
|---|---|---|---|
| Paclitaxel | Teva | 7 µM | 2,5-5-10-15-30-50 nM |
| *Cisplatin* | Pharmacia Nostrum, S.A. DG0543 | 3,3 nM | 2-5-10-20-30-50 µM |
| *5-Fluorouracil* | Accord Healthcare, S.L.U. PY07286 | 0.384 M | 0,625-2,5-10-20-40-80 µg/ml |

The viability percentage was calculated by subtracting the blank and normalizing the control measurement as 100%. For measurement at each time point, 10 µL of the MTT compound (1 mg/mL) was added to each well and incubated at 37°C for 1 hour. Afterwards, the medium was aspirated, and 100 µL of DMSO was added, shaking the plates for 5 minutes to dissolve the formazan crystals. The absorbance of the samples was read by the UltraEvolution microplate reader (Tecan^{®}) at 570 nm. For each drug, the assay was performed in duplicate.

### Example 1.10.2. In vitro cell proliferation assay

Cell proliferation of the clones was determined by measuring cell viability following the previously explained MTT protocol at 0, 24, 48 and 72 hours after seeding 2000 cells/well in a 96- well plate. A blank measure (without cells) was included. The data at each time-point was calculated by subtracting the blank and normalizing the measurements at 0 hours as 1.

### Example 1.11. Migration assay

The study of changes in the migratory capacity after MED 12 alteration was conducted through the wound closure assay that consists of observing the migration of a monolayer of cells as they establish new cell-cell contacts to close a "wound" previously created in the culture plate. For each condition, 350,000 cells were seeded per well in a 6-well plate and allowed to culture for 48 hours until a full monolayer (100% confluence) was formed. Once this was achieved, the medium was replaced with RPMI medium without FBS to arrest cell proliferation, a key step to prevent interference with the results. After 24 hours cells were washed once with PBS. New medium without FBS was added and 2 vertical "scratches" were made in each well with a 10 µL tip. At this point, image recording by NIKON ECLIPSE TE-200-E phase-contrast microscope was initiated. Photos were taken of 2 regions of each scratch every 30 minutes until the wound was fully closed, approximately 22 hours later.

To determine the migration percentage, the ImageJ^{®} software was used to process the images captured under the microscope. Specifically, the MRI Wound Healing Tool was employed to measure the free area of the wound at each time-point. The reduction in the free area over time for each of the 4 replicates of each condition allowed the calculation of the occupied area percentage, and therefore, the migration speed.

### Example 1.12. Statistical analysis

The statistical analysis was conducted using the PRISM10 software. Statistical significance was concluded for P ≤ 0.05 using the student's t-test for comparing the three different conditions with the control, independently. The normality of the data was previously confirmed with Kolmogorov-Smirnov when n>50 and Shapiro-Wilk test when n<50. For this test, normal distribution was assumed when p > 0.05. All graphics included in this work were obtained with this same PRISM10 software.

### Example 2. Results

### Example 2.1. CRISPR/Cas9-Mediated MED12 Editing in the 32816 Cell Line

Following the transfection of the 32816-cell line with a CRISPR/Cas9 construct designed to inactivate MED12, a total of 30 MED12-edited clones and 30 control clones were isolated by single-cell sorting (**Table 7**). These clones were expanded in 24-well and 6-well plates and subsequently cultured in Petri dishes.

**Table 7. Analysis of CRISPR/CAS9 gene-editing system efficiency.**

| Condition | Transfection Efficiency | Single Cell | Clones Generated | Edited-Clones Validated |
|---|---|---|---|---|
| Empty vector | 21 % GFP + | 1 plate of 96-well | 17 | 0 |
| G1 + G2 | 30 % GFP + | 1 plate of 96-well | 16 | 7 |

Validation of gene editing at the DNA level was conducted using the CRISPR/Cas9 sgRNAs (**Table 2**) as primers for qPCR. The results showed that 7 clones had their oligonucleotide sequences edited at the targeted region, as indicated by the absence of amplification. (**Figure 1**). The other 9 clones showed amplification similar to the control, resulting in a CRISPR/Cas9 efficiency of 43.75%.

From this point, three successfully MED12-edited clones were selected for further characterization. Also, three clones that had been transfected with the empty plasmid were used as controls in all the subsequent experiments. PCR analysis of DNA samples from all clones, using primers targeting MED12, resulted in the amplification of a full-length fragment (412 pb) in the control clones. However, the edited clones showed shorter fragment (**Figure 2A**). Consistently, Sanger sequencing of these PCR products revealed homozygotic deletions within N-terminal of the MED12 gene, near the sgRNA target region. Noted, the number of pair of bases (pb) loss and specific position differed in each clone.

Protein expression predictions based on these results indicated that whereas the first clone presents a disruption in the protein expression (MED12-Knockout, KO), the other two are still able to produce an aberrant protein (MED12-Mutated, Mut) (**Table 8**). These predictions were indeed confirmed by western blot analysis (**Figure 2B**). Moreover, the morphology among the generated cell lines also differed. The MED12-Mut ones resembled the wildtype (WT) cell line, whereas the MED12-KO clones exhibited noticeable changes. The most prominent difference observed in the MED12-KO cells was their growth pattern, which formed clumps (**Figure 3**).

**Table 8. Predicted outcomes by VarSome regarding protein expression.**

| Clone Number | gDNA alteration | Protein Outcome | Protein Effect | Clon Name for Further Experiments |
|---|---|---|---|---|
| Control | None | No changes | Normal Expression | Control |
| *1* | c,120_121del (2bp). Hom. | p.(Val41LysfsTer6) | Disruption of Expression | *MED12*-KO |
| | c.125_170del (46bp). Hom. | p.(Lys42ThrfsTer40) | | |
| 2 | c.125_127del (3bp); Hom. | p.(Gln43del) | Expression of an Aberrant Protein | *MED12*-Mut.1 |
| | c.137_160del (24bp); Hom. | p.(Asn47_Asp54del) | | |
| *3* | c.121_123del (3bp) | p.(Val41del) | Expression of an Aberrant Protein | *MED12*-Mut.2 |

Importantly, during western blot analysis we encountered a worth-mentioned challenge regarding CRISPR/Cas9-edition validation. The successful validation of CRISPR/Cas9-edition showed in **Figure 2B** was performed with and antibody targeting the N-terminal region of the protein. However, when using another available antibody targeting the C-terminal, protein expression in all conditions was observed (**Figure 4A**). These results were inconsistent, revealing a specificity issue with this second antibody.

Next, we performed a bibliographic research and saw that C-terminal domain of MED 12 shares total conservation with its homolog MED12-L, suggesting a potential cross-reaction in the MED12-KO cells. Subsequent analysis via NCBI blast confirmed MED12-L protein recognition as an off-target, as depicted in **Figure 4B****.** The next step was to confirm the presence of endogenous wildtype MED12-L in our clones. For this purpose, PCR from cDNA was performed, showing amplification for MED12-L in all cases with amplicon sizes identical to control clones, suggesting that no edition occurred for this gene. Thus, our sgRNAs CRISPR/Cas9 guides must be specific for MED12.

Taken together, these results confirmed again the MED 12 editing and the predicted protein outcomes, proving the success of our CRISPR/Cas9-mediated clone generation. Moreover, it proves the presence and non-edition of MED12-L in our models, suggesting a cross-reaction of the antibody targeting the C-terminal of these 2 homologous that would lead to the recognition of both indistinctly.

### Example 2.2. Functional Characterization of the Alteration of MED12 in HNSCC

### Example 2.2.1. Study of Cell Proliferation

The proliferation assay conducted with the selected clones revealed decreased proliferation over 72 hours for all three MED12-edited clones compared to the average of the three control clones included in the study. **Figure 5** presents the proliferation rate of each clone along with their respective standard deviations, showing significant differences (p < 0.05) in all cases. Surprisingly, MED12-Mut-1 and Mut.2 clones showed the same phenotype as the KO one despite still expressing MED12 protein. Both present alterations within the N-terminal of the protein. Therefore, our results suggest a strong link between this specific region and cell proliferation.

Furthermore, there is a known association between MED12-related alterations and CDK8 function regarding this biological process. Because of this, we decided to conduct immunofluorescence analysis to evaluate potential changes in the expression or cellular localization of the kinase. As shown in **Figure 6** no significant differences in expression levels were found. However, the results showed a slight reduction of CDK8 in the cytoplasm and a more pronounced focus in the nuclei when MED12 expression is altered. For the MED12-KO condition, assessing cytoplasmatic localization differences was challenging due to its growth in clumps, with no conclusive results.

### Example 2.2.2. Study of Cell Migration

Next, we aimed to determine if N-terminal alteration of MED12 could also affect cell migration. To assess this, we conducted a wound healing assay, analyzing four replicated per clone. Cells lacking MED12 expression demonstrated a higher migratory capacity at all time points, as the percentage of occupied area was significantly greater than the control condition (p-value <0.001). However, no differences were found between the MED12-mut clones and the controls.

All wounds were nearly closed within 6 hours when MED12 was inactivated, while both the control and the MED12-mut clones did not fully close even 22 hours after the wound was made, at which point imaging ceased. (**Figure 7A**). Detailed percentages of the occupied area for each replicate and time point selected for the statistical analysis are shown in **Figure 7B****.**

### Example 2.3. Response to Conventional HNSCC Treatment

Considering our proliferation assay results, we investigated whether the therapeutic response of our models was altered, as reduced proliferation rates are often associated with treatment resistance. Therefore, independent cytotoxic assays were conducted with three drugs included in the conventional TPF regimen, routinely administered to HNSCC patients. The selected clones were treated with increasing doses of cisplatin, paclitaxel, and 5-fluorouracil. The time points for measuring cellular viability depended on the specific action mechanism of each drug: cisplatin and paclitaxel exhibit early effects as soon as 24 hours after administration, while the effects of 5-FU are not observable until 48 hours later. The graphs obtained from the assay are presented in **Figure 8****.** Both MED12-edited and control clones exhibited a dose and time-dependent response to each of the drugs. Although significant p-values were observed at some specific points, especially in the response to cisplatin of the MED12-KO cells, the results indicated no overall differences in the general response to the TPF drugs.

### Example 2.4. Preliminary results on shRNA-Mediated MED12 Silencing in the 32816 Cell Line

Given the potential of inactivating MED12 protein function that our previous results suggest, we decided to establish an shRNA model to complement our CRISPR findings and provide another robust tool for studying MED12 function. The electroporation efficiency of the 32816-cell line with three different shRNA sequences after 48 hours is detailed in **Table 9.** Surprisingly, no synergic effect was observed when the three shRNA were transfected combined. Neomycin selection concluded within seven days, resulting in four viable populations of potentially silenced cells and one control population.

**Table 9. Analysis of MED12-shRNAtransfection efficiency.**

| | Empty vector | *MED12*-sh1 | *MED12*-sh2 | *MED12*-sh3 | *MED12*-sh1-2-3 |
|---|---|---|---|---|---|
| Transfection Efficiency | Unknown. The vector does not have EGFP gene. | 30.3% GFP+ | 6.6.% GFP+ | 15.8% GFP+ | 19% GFP+ |

Validation of protein silencing was conducted via Western blot analysis. The results revealed that while sh1 did not reduce MED12 expression compared to the control, sh2, sh3 and the combination of the three effectively silenced the gene, with protein expression being completely disrupted. Again, when testing the antibody targeting the C-terminal, protein levels were detected, confirming the cross-reaction with MED12L. (**Figure 9A**). Consistently, cells transfected with these shRNAs exhibited growth patterns similar to the previously described in MED12 knockout (KO) cells, characterized by clumping (**Figure 9B**). These results validate this specific shRNA-mediated silencing approach as an effective alternative to CRISPR/Cas9 for inactivating MED 12 expression and for monitoring the effect of MED 12 inactivation on cell behaviour in future studies.

## Claims

1. MED12 inhibitor, or pharmaceutical composition comprising thereof, for use in a method for the treatment of cancer.

2. MED12 inhibitor, or pharmaceutical composition comprising thereof, for use, according to claim 1, **characterized in that** the inhibitor is a genetic inhibitor designed to interfere with the expression or function of MED 12 gene.

3. MED12 inhibitor, or pharmaceutical composition comprising thereof, for use, according to any of the previous claims, **characterized in that** the inhibitor is shRNA, siRNA, CRISPR-Cas9 or anti-MED12 antibodies.

4. MED12 inhibitor, or pharmaceutical composition comprising thereof, for use, according to any of the previous claims, **characterized in that** the inhibitor is a small molecule inhibitor.

5. MED12 inhibitor, or pharmaceutical composition comprising thereof, for use, according to any of the previous claims, in combination with chemotherapy.

6. MED12 inhibitor, or pharmaceutical composition comprising thereof, for use, according to any of the previous claims, in a method for the treatment of solid tumours comprising head and neck or ovarian cancer.

7. Combination drug product comprising a MED12 inhibitor and chemotherapy.

8. Combination drug product, according to claim 7, wherein the inhibitor is a genetic inhibitor designed to interfere with the expression or function of MED 12 gene.

9. Combination drug product, according to any of the claims 7 or 8, wherein the inhibitor is shRNA, siRNA, CRISPR-Cas9 or anti-MED12 antibodies.

10. Combination drug product, according to any of the claims 7 to 9, wherein the inhibitor is a small molecule inhibitor.

11. Pharmaceutical composition comprising the combination drug product of claims 7 to 10 and, optionally, pharmaceutically acceptable excipients or carriers.

12. Combination drug product, according to any of the claims 7 to 10, or pharmaceutical composition, according to claim 11, for use in a method for the treatment of cancer.

13. Combination drug product, according to any of the claims 7 to 10, or pharmaceutical composition, according to claim 11, for use, according to claim 12, in a method for the treatment of solid tumours comprising head and neck or ovarian cancer.

14. *In vitro* method for screening, identifying and/or producing compounds for use in the treatment of cancer which comprises: a) Assessing MED12 gene expression once the candidate compound has been incubated with MED12 gene, and b) wherein if an inhibition of MED12 gene expression is observed, it is indicative that the candidate compound may be effective for use in the treatment of cancer.

15. *In vitro* method, according to claims 14, for screening, identifying and/or producing compounds for use in the treatment of head and neck or ovarian cancer, which comprises: a) Assessing MED12 gene expression once the candidate compound has been incubated with MED12 gene, and b) wherein if an inhibition of MED12 gene expression is observed, it is indicative that the candidate compound may be effective for use in the treatment of head and neck or ovarian cancer.
